(19) Europäisches Patentamt
European Patent Office
Office européen des brevets

(11) Publication number: 0 112 821 B1

(12) # EUROPEAN PATENT SPECIFICATION

(45) Date of publication of patent specification: 16.07.86

(51) Int. Cl.[4]: C 07 C 45/45

(21) Application number: 82902338.1

(22) Date of filing: 25.06.82

(86) International application number:
PCT/US82/00863

(87) International publication number:
WO 84/00160 19.01.84 Gazette 84/02

(54) **PROCESS FOR PREPARING SATURATED DIMERIC KETONES.**

(43) Date of publication of application:
11.07.84 Bulletin 84/28

(45) Publication of the grant of the patent:
16.07.86 Bulletin 86/29

(84) Designated Contracting States:
BE DE FR GB NL

(56) References cited:
BE-A- 623 186
CA-A- 665 765
DE-A-1 936 203
FR-A-2 020 577
JP-A-41 004 966
US-A-3 361 828
US-A-3 574 763
US-A-3 953 517
US-A-3 966 822
US-A-4 011 278
US-A-4 049 571

The file contains technical information submitted after the application was filed and not included in this specification

(73) Proprietor: MOBIL OIL CORPORATION
150 East 42nd Street
New York New York 10017 (US)

(72) Inventor: HUANG, Tracy J.
9 Woodfield Lane
Lawrenceville, NJ 08648 (US)
Inventor: HAAG, Werner O.
38 Pine Knoll Drive
Lawrenceville, NJ 08648 (US)

(74) Representative: West, Alan Harry
Mobil Court 3 Clements Inn
London WC2A 2EB (GB)

(56) References cited:
US-A-4 102 930
US-A-4 170 609
US-A-4 270 006

Chemical Abstracts, vol. 84, no. 5, issued 02 February 1976, Columbus, Ohio, US, abstract no. 30550f, A.D.Guseinov "Condensation of acetone or crystalline alumino-silicates", Nef.Gay., 1974, pp. 146-7 (Russ)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 112 821 B1

(56) References cited:
**Chemical Abstracts, vol. 79, no. 15, issued 15 October 1973, Columbus, Ohio, US, abstract no. 916966, O'Kyznetsov, "Condensation of acetone on synthetic zeolite", Neftepererab Neftekhim 1073 (3), 289, (Russ)**

**Description**

This invention relates to a process for making saturated dimeric ketones using a zeolite catalyst.

Current commercial processes for converting monomeric ketones into saturated dimeric ketones require three synthesis steps, each of which has a relatively low yield. Such processes can be exemplified by the conversion of acetone into methylisobutyl ketone (MIBK) as follows:

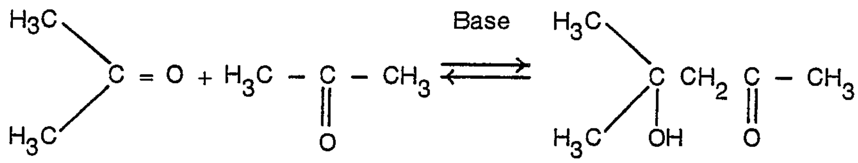


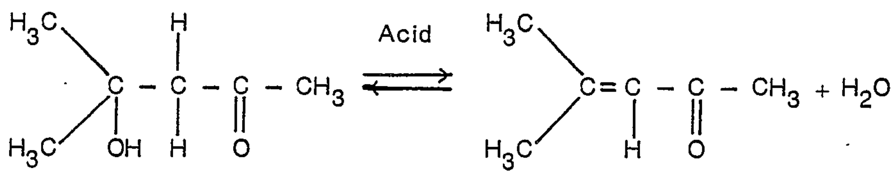


Mesityl Oxide

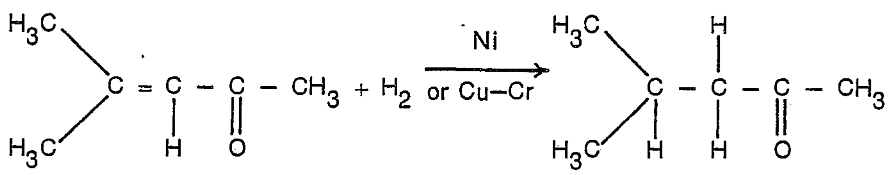


MIBK

The mesityl oxide prepared in the second step is separated and hydrogenated in the third step over Ni or Cu-Cr catalyst to form MIBK.

In this synthesis, the following side reactions also take place:

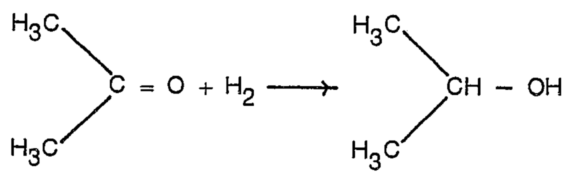

Isopropyl Alcohol

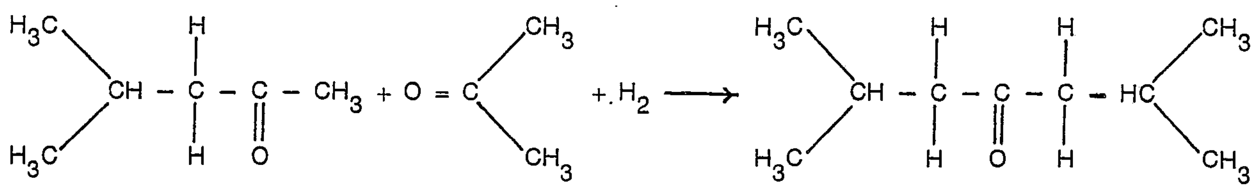

Diisobutyl Ketone

The formation of diisobutyl ketone is a result of further condensation of MIBK with acetone. Heavier ketones, such as $C_{12}$ ketone, have also been observed in a Pd/resin bifunctional system (see German Patent 1,260,454).

Direct synthesis of methylisobutyl ketone from acetone over bifunction catalysts, for example Pd/faujasite (Japanese Patents 46—2009 and 46—2643, DE—A—1,936,203 and FR—A,2,020,577), Pd/cation exchange resin (German Patent 1,260,454), KOH-alumina-Pd (U.S. Patent 2,499,172), and MgO-silica-Pd (British Patent 1,015,003), has also been reported.

U.S. Patent 3,998,898 discloses that acetone can be converted to mesitylene by passing it over an acid catalyst, for example an acid zeolite. However, that patent neither discloses nor suggests that a Group VIII metal-containing zeolite will selectively yield the dimeric ketone in superior amounts.

In accordance with the present invention, there is provided a process for preparing a saturated dimeric ketone of the formula

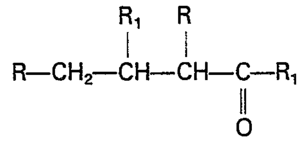

in which each R is hydrogen or a $C_1$—$C_5$ alkyl group and each $R_1$ is a $C_1$—$C_6$ alkyl group, comprising contacting hydrogen and a ketone of the formula

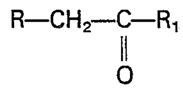

in which R and $R_1$ have the meanings given above with a crystalline zeolite having the formula, expressed in terms of mole ratios of its constituent oxides, as follows:

$$\leq 1.3M_{\frac{2}{n}}O:W_2O_3:xYO_2:zH_2O$$

in which M is a cation of valence n, W is one or more trivalent metal atoms from Groups III to VIII of the Periodic Table, Y is silicon or germanium, x is greater than 5 and z is 0 to 40, and containing a group VIII metal incorporated in the zeolite by exchange, impregnation or physical admixture.

In the above formula, W may be a Group IIIB metal, for example aluminum, gallium, boron; a Group VIA metal, for example chromium; or a Group VIII metal, for example iron, and x is preferably greater than 12.

In a preferred synthesized form, the zeolite has a formula, expressed in terms of mole ratios of its constituent oxides, as follows:

$$\leq 1.3M_{\frac{2}{n}}O:Al_2O_3:xSiO_2:zH_2O$$

in which M is a mixture of alkali metal cations, especially sodium, and alkylammonium cations the alkyl groups of which preferably contain from 2 to 5 carbon atoms, and x is greater than 12.

It is especially preferred that the zeolite is an aluminosilicate zeolite (in which case W is aluminum and Y is silicon) having a silica/alumina mole ratio of at least 12 but that can range up to 4000 or more, and a Constraint Index of 1 to 12.

The term "zeolite" is used generally herein to define natural or synthetic porous tectosilicates characterized by having a rigid crystalline framework structure composed of an assembly of silicon atoms and at least a trace amount of a trivalent metal atom, for example iron, boron, gallium, chromium and the like or mixtures thereof, but preferably aluminum, the silicon atoms and trivalent metals each being surrounded by a tetrahedron of shared oxygen atoms, and a precisely defined pore structure.

The crystalline zeolites utilized herein are members of a class of zeolitic materials which exhibit unusual properties. Although these zeolites have unusually low alumina contents, i.e. high silica to alumina mole ratios, they are very active even when the silica to alumina mole ratio exceeds 30. The activity is surprising since catalytic activity is generally attributed to framework aluminum atoms and/or cations associated with these aluminum atoms. These zeolites retain their crystallinity for long periods in spite of the presence of steam at high temperature which induces irreversible collapse of the framework of other zeolites, e.g. of the X and A type. Furthermore, carbonaceous deposits, when formed, may be removed by burning at higher than usual temperatures to restore activity. These zeolites, used as catalysts, generally have low coke-forming activity and therefore are conducive to long times on stream between regenerations by burning carbonaceous deposits with oxygen-containing gas such as air.

An important characteristic of this class of zeolites is that the crystal structure provides a selective constrained access to and egress from the intracrystalline free space by virtue of having an effective pore size intermediate between the small pore Linde A and the large pore Linde X, i.e. the pore windows of the structure are of about a size such as would be provided by 10-membered rings of silicon atoms interconnected by oxygen atoms. It is to be understood, of course, that these rings are those formed by the regular disposition of the tetrahedra making up the anionic framework of the cyrstalline zeolite, the oxygen atoms themselves being bonded to the silicon (or aluminum, etc.) atoms at the centers of the tetrahedra.

The silica to alumina mole ratio referred to may be determined by conventional analysis. This ratio is meant to represent, as closely as possible, the ratio in the rigid anionic framework of the zeolite crystal and to exclude aluminum in the binder or in cationic or other form within the channels. Although zeolites with a

silica to alumina mole ratio of at least 12 are useful, it is preferred in some instances to use zeolites having substantially higher silica/alumina ratios, e.g. 1600 and above. In addition, zeolites as otherwise characterized herein but which are substantially free of aluminum, that is zeolites having silica to alumina mole ratios of up to infinity, are found to be useful and even preferable in some instances. Such "high silica" or "highly siliceous" zeolites are intended to be included within this description. Also to be included within this definition are substantially pure silica analogs of the useful zeolites described herein, that is to say those zeolites having no measurable amount of aluminum (silica to alumina mole ratio of infinity) but which otherwise embody the characteristics disclosed.

This class of zeolites, after activation, acquire an intracrystalline sorption capacity for normal hexane which is greater than that for water, i.e. they exhibit "hydrophobic" properties. This hydrophobic character can be used to advantage in some applications.

The class of zeolites useful herein have an effective pore size such as to freely sorb normal hexane. In addition, the structure must provide constrained access to larger molecules. It is sometimes possible to judge from a known crystal structure whether such constrained access exists. For example, if the only pore windows in a crystal are formed by 8-membered rings of silicon and aluminum atoms, then access by molecules of larger cross-section than normal hexane is excluded and the zeolite is not of the desired type. Windows of 10-membered rings are preferred, although in some instances excessive puckering of the rings or pore blockage may render these zeolites ineffective.

Although 12-membered rings in theory would not offer sufficient constraint to produce advantageous conversions, it is noted that the puckered 12-ring structure of TMA offretite does show some constrained access. Other 12-ring structures may exist which may be operative for other reasons and, therefore, it is not the present invention to entirely judge the usefulness of a particular zeolite solely from theoretical structural considerations.

Rather than attempt to judge from crystal structure whether or not a zeolite possess the necessary constrained access to molecules of larger cross-section than normal paraffins, a simple determination of the "Constraint Index" may be used. The meaning of Constraint Index and its method of determination are fully described in for example, U.S. Patent 3,905,915.

The preferred class of zeolites used herein are ZSM—5, ZSM—11, ZSM—12, ZSN—23, ZSM—35, ZSM—38, ZSM—48 and other similar materials; ZSM—5 is especially preferred.

ZSM—5 is described in U.S. Patents Nos. 3,702,886 and Re 29,948. ZSM—11 is described in U.S. Patent No. 3,709,979. ZSM—12 is described in U.S. Patent No. 3,832,449. ZSM—23 is described in U.S. Patent No. 4,076,842. ZSM—35 is described in U.S. Patent No. 4,016,245. ZSM—38 is described in U.S. Patent No. 4,046,859. ZSM—48 is described in published European Patent Application No. 15132.

These specific zeolites, when prepared in the presence of organic cations, are substantially catalytically inactive, possibly because the intra-crystalline free space is occupied by organic cations from the forming solution. They may be activated by heating in an inert atmosphere at 540°C for one hour, for example, followed by base exchange with ammonium salts followed by calcination at 540°C in air. The presence of organic cations in the forming solution may not be absolutely essential to the formation of this type of zeolite; however, the presence of these cations does appear to favor the formation of this class of zeolite. More generally, it is desirable to activate this type catalyst to base exchange with ammonium salts followed by calcination in air at about 540°C for from about 15 minutes to about 24 hours.

Natural zeolites may sometimes be converted to zeolite structures of the class herein identified by various activation procedures and other treatments such as base exchange, steaming, alumina extraction and calcination, alone or in combinations. Natural minerals which may be so treated include ferrierite, brewsterite, stilbite, dachiardite, epistilbite, heulandite, and clinoptilolite.

As an additional feature, the zeolites preferably have also a crystal framework density, in the dry hydrogen form, of not less than 1.6 grams per cubic centimeter. The dry crystal density for known structures may be calculated from the number of silicon plus aluminum atoms per 1000 cubic Angstroms, as described on page 19 of the article ZEOLITE STRUCTURE by W. M. Meier, included in PROCEEDINGS OF THE CONFERENCE ON MOLECULAR SIEVES, (London, April 1967) published by the Society of Chemical Industry, London, 1968.

When the crystal structure is unknown, the crystal framework density may be determined by classical pyknometer techniques. For example, it may be determined by immersing the dry hydrogen form of the zeolite in an organic solvent which is not sorbed by the crystal. Alternatively the crystal density may be determined by mercury porosimetry, since mercury will fill the interstices between crystals but will not penetrate the intracrystalline free space.

When synthesized in the alkali metal form, the zeolite is conveniently converted to the hydrogen form, generally by intermediate formation of the ammonium form as a result of ammonium ion exchange and calcination of the ammonium form to yield the hydrogen form. In addition to the hydrogen form, other forms of the zeolite wherein the original alkali metal has been reduced to less than about 1.5 percent by weight may be used. Thus, the original alkali metal of the zeolite may be replaced by ion exchange with other suitable metal cations of Groups I through VIII of the Periodic Table, including for example, nickel, copper, zinc, palladium, calcium or rare earth metals.

In practicing a particularly desired chemical conversion process, it may be useful to incorporate the above-described crystalline zeolite with a matrix comprising another material resistant to the temperature

and other conditions employed in the process. Such matrix material is useful as a binder and imparts greater resistance to the catalyst for the severe temperature, pressure and reactant feed stream velocity conditions encountered in many processes.

Useful matrix materials include both synthetic and naturally occurring substances, as well as inorganic materials such as clay, silica and/or metal oxides. The latter may be either naturally occurring or in the form of gelatinous precipitates or gels including mixtures of silica and metal oxides. Naturally occurring clays which can be composited with the zeolite include those of the montmorillonite and kaolin families, which families include the sub-bentonites and the kaolins commonly known as Dixie, McNamee-Georgia and Florida clays or others in which the main mineral constituent is halloysite, kaolinite, dickite, nacrite or anauxite. Such clays can be used in the raw state as originally mined or initially subjected to calcination, acid treatment or chemical modification.

In addition to the foregoing materials, the zeolites employed herein may be composited with a porous matrix material, such as alumina, silica-alumina, silica-magnesia, silica-zirconia, silica-thoria, silica-beryllia, and silica-titania, as well as ternary compositions, such as silica-alumina-thoria, silica-alumina-zirconia, silica-alumina-magnesia and silica-magnesia-zirconia. The matrix may be in the form of a cogel. The relative proportions of zeolite component and inorganic oxide gel matrix, on an anhydrous basis, may vary widely with the zeolite content ranging from between about 1 to about 99 percent by weight and more usually in the range of about 5 to about 80 percent by weight of the dry composite.

The process of the invention is carried out in the presence of a catalyst comprising the zeolite and a Group VIII metal. Preferred Group VIII metals are Pd, Pt, Ru, Rh, Ir and Ni, Pd being especially preferred. A particularly preferred Group VIII metal/zeolite combination is Pd/ZSM—5.

Preferred ketones used in the process of the present invention are those in which R is hydrogen and $R_1$ is methyl or ethyl, that is dimethyl ketone and methyl ethyl ketone. Other ketones that may be used include methylbutyl ketone, methylhexyl ketone, diethyl ketone, dipropyl ketone, butyl hexyl ketone and amylhexyl ketone.

The process is carried out at a temperature of from 100°C to 350°C, at a pressure of from 0 to 2000 psig (100 to 14,000 kPa), and for a contact time sufficient to achieve the desired degree of ketone conversion. If as is preferred, the process is carried out in a fixed bed flow reactor, the weight hourly space velocity (WHSV) is from about 0.2 to about 20. The metal content of the catalyst is suitably from 0.01 to 2.0 weight percent, preferably from 0.05 to 0.5 weight percent. The metal incorporation can be carried out either by impregnation or ion exchange. Alternatively, a physical mixture of zeolite and metal catalyst components can be used. The hydrogen/ketone ratio can vary from 0.1 to 5.0. The preferred process conditions are: temperature — from 150°C to 240°C; pressure — from 400 to 1000 psig (2,900 to 7,000 kPa); WHSV — from 1 to 8; and $H_2$/ketone mole ratio — from 0.4 to 1.0.

As has been stated above, the zeolites used in the process of the invention may have the original cations associated therewith wholly or partly replaced by a wide variety of other cations according to tehniques well known in the art, by ion exchange. Typical replacing cations include hydrogen, ammonium, and metal cations. Of the replacing cations, particular preference is given to cations of hydrogen, alkali, ammonium, rare earth, magnesium, calcium, zinc, copper, silver, platinum, palladium, nickel and mixtures thereof. The metals may be also added by impregnation.

Typical ion exchange techniques include contacting the particular zeolite with a salt of the desired replacing cation or cations. Although a wide variety of salts can be employed, particular preference is given to chlorides, nitrates and sulfates. Pd and Pt can also be exchanged via their tetramine complex ions.

Representative ion exchange techniques are disclosed in a wide variety of patents, including U.S. Patents 3,140,249; 3,140,251; and 3,140,253.

Following contact with the salt solution of the desired replacing cation, the zeolites may be washed with water and dried at a temperature of from 65 to 315°C and thereafter may be heated in air or other inert gas at temperatures ranging from 260 to 930°C for periods of time ranging from 1 to 48 hours or more.

The following examples illustrate the invention.

Example 1

Single-step synthesis of methylisobutyl ketone over Pd/ZSM—5

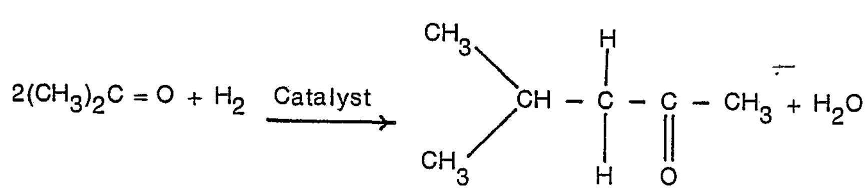


A sample of ZSM—5 containing 0.5 percent by weight of Pd was placed in a stainless steel reactor and the unit was pressurized with helium to 600 psig (4,200 kPa) and heated to 180°C. After the desired temperature was reached, actone, together with hydrogen, was fed into the reactor at a $H_2$/acetone mole ratio of 0.6 and an acetone weight hourly space velocity (WHSV) of 3.8. The conversion was conducted in a

fixed-bed reactor in down-flow fashion. The products were collected and analyzed by gas chromatography. The results showed that acetone conversion was 28.5 weight percent and MIBK selectivity was 98.3 weight percent.

Example 2

The procedure of Example 1 was repeated except that a sample of REY (rare earth-containing faujasite Y) containing 0.35 percent Pd was used as catalyst. This catalyst resulted in only 30 weight percent MIBK selectivity with an acetone conversion of 29.4 weight percent.

The results obtained in Examples 1 and 2 are set out in the following Table:

| | Example 1 | Example 2 |
|---|---|---|
| Catalyst | Pc/ZSM—5 | Pd/REY |
| Temperature | 180°C | 180°C |
| Pressure | 600 psig (4,200 kPa) | 600 psig (4,200 kPa) |
| WHSV (Acetone) | 3.8 | 3.8 |
| $H_2$/Acetone (mole ratio) | 0.6 | 0.6 |
| Reactor Effluent, weight percent | | |
| Diisopropyl Ether | — | 3.6 |
| Acetone | 71.5 | 70.6 |
| Isopropyl Alcohol | 0.4 | 14.2 |
| Mesityl Oxide | — | 0.1 |
| MIBK | 23.8 | 8.1 |
| $C_9$ Ketones* | — | 1.0 |
| Water | 4.3 | 2.4 |
| Acetone Conversion, weight percent | 28.5 | 29.4 |
| Product Selectivity, **weight percent | | |
| Diisopropyl ether | — | 13.3 |
| Isopropyl Alcohol | 1.7 | 52.6 |
| Mesityl Oxide | — | 0.4 |
| MIBK | 98.3 | 30.0 |
| $C_9$ Ketones* | — | 3.7 |

* Mainly diisobutyl ketone
** Excluding water

Comparison of the results given in the Table clearly demonstrates that Pd/ZSM—5 gives a MIBK selectivity advantage over Pd/REY. As can be seen further, the Pd/ZSM—5 catalyst produces no diisobutyl ketone.

**Claims**

1. A process for preparing a saturated dimeric ketone of the formula

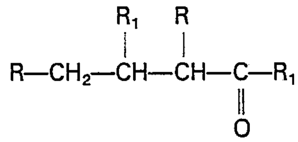

in which each R is hydrogen or a $C_1$—$C_5$ alkyl group and each $R_1$ is a $C_1$—$C_6$ alkyl group, comprising contacting hydrogen and a ketone of the formula

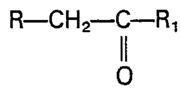

in which R and $R_1$ have the meanings given above, with a crystalline zeolite having the formula, expressed in terms of mole ratios of its constituent oxides, as follows:

$$\leq 1.3M_{\frac{2}{n}}O:W_2O_3:xYO_2:zH_2O$$

in which M is a cation of valence n, W is a trivalent metal atom from Groups III to VIII of the Periodic Table, Y is silicon or germanium, x is greater than 5 and z is 0 to 40, and containing a group VIII metal incorporated in the zeolite by exchange, impregnation or physical admixture, the process being carried out at 100 to 300°C, from 0 to 2000 psig (100 to 14,000 kPa) and a weight hourly space velocity of 0.2 to 20.

2. A process according to Claim 1, wherein the ketone is acetone and the product is methylisobutyl ketone.

3. A process according to Claim 1 or Claim 2, wherein the Group VIII metal is palladium.

4. A process according to any one of Claims 1 to 3, wherein the metal content of the catalyst is from 0.01 to 2.0 weight percent.

5. A process according to any one of Claims 1 to 4, wherein the zeolite is an aluminosilicate zeolite.

6. A process according to Claim 5, wherein the aluminosilicate zeolite has a silica/alumina mole ratio of greater than 12 and a Constraint Index of 1 to 12.

7. A process according to Claim 6, wherein the zeolite is ZSM—5.

8. A process according to Claim 7, wherein the catalyst is a physical mixture of ZSM—5 and supported palladium.

**Patentansprüche**

1. Verfahren zum Herstellen eines gesättigten dimeren Ketons der Formel

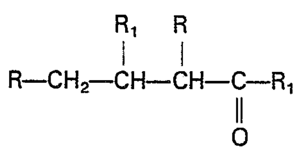

in der jeder Rest R ein Wasserstoffatom oder einen $C_1$—$C_5$-Alkylrest und jeder Rest $R_1$ einen $C_1$—$C_6$-Alkylrest bedeutet, dadurch gekennzeichnet, daß man Wasserstoff und ein Keton der Formel

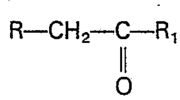

in der R und $R_1$ die oben angegebenen Bedeutungen haben, mit einem kristallinen Zeolith der folgenden Formel, ausgedrückt in Molverhältnissen der ihn aufbauenden Oxide,

$$\leq 1.3M_{\frac{2}{n}}O:W_2O_3:xYO_2:zH_2O$$

in Kontakt bringt, worin M ein Kation mit der Wertigkeit n, W ein dreiwertiges Metallatom der Gruppen III bis VIII des Periodensystems, Y Silicium oder Germanium, x eine Zahl größer als 5 und z eine Zahl von 0 bis 40 bedeuten, und worin ein Metall der Gruppe VIII enthalten ist, das in den Zeolithen durch Austausch, Imprägnieren oder physikalisches Mischen eingebracht worden ist, wobei das Verfahren bei 100 bis 300°C, 0 bis 2000 psig (100 bis 14.000 kPa) und einer auf das Gewicht bezogenen stündlichen Raumgeschwindigkeit von 0,2 bis 20 durchgeführt wird.

2. Verfahren nach Anspruch 1, worin das Keton Aceton und das Produkt Methylisobutylketon ist.

3. Verfahren nach Anspruch 1 oder 2, worin das Metall der Gruppe VIII Palladium ist.

4. Verfahren nach einem der Ansprüche 1 bis 3 worin der Metallgehalt des Katalysators 0,01 bis 2,0 Gew.-% beträgt.

5. Verfahren nach einem der Ansprüche 1 bis 4, worin der Zeolith ein Aluminiumsilicat-Zeolith ist.

6. Verfahren nach Anspruch 5, worin der Aluminiumsilicat-Zeolith ein Siliciumdioxid-Aluminiumoxid Molverhältnis von über 12 und einen Constraint-Index von 1 bis 12 aufweist.

7. Verfahren nach Anspruch 6, worin der Zeolith ein ZSM—5 ist.

8. Verfahren nach Anspruch 7, worin der Katalysator ein physikalisches Gemisch aus ZSM—5 und auf einem Träger befindlichen Palladium ist.

**Revendications**

1. Procédé pour la fabrication d'une cétone dimère saturée de formule

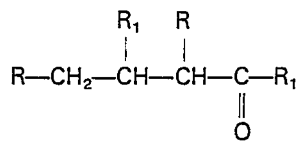

dans laquelle chaque reste R est l'hydrogène ou un groupe alkyle en $C_1$—$C_5$ et chaque reste $R_1$ est un groupe alkyle en $C_1$—$C_6$, comprenant la mise en contact d'hydrogène et d'une cétone de formule

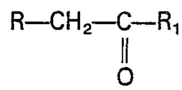

dans laquelle R et $R_1$ ont les significations indiquées ci-dessus avec une zéolite cristalline ayant la formule suivante, exprimée en rapports molaires des oxydes la constituant:

$$\leq 1.3 M_{\frac{2}{n}}O : W_2O_3 : xYO_2 : zH_2O$$

dans laquelle M est un cation de valence n, W représente un ou plusieurs atomes de métaux trivalents des groupes III à VIII de la Classification Périodique, Y représente le silicium ou le germanium, x est supérieur à 5 et z est un nombre de 0 à 40, et contenant un métal du groupe VIII incorporé dans la zéolite par échange, imprégnation ou mélange physique, le procédé étant mis en oeuvre à 100—300°C, sous une pression de 0 à 2000 psig (100 à 14 000 kPa) et avec un vitesse spatiale horaire en poids de 0,2 à 20.

2. Procédé selon la revendication 1, dans lequel la cétone est l'acétone et le produit est la méthylisobutylcétone.

3. Procédé selon la revendication 1 ou 2, dans lequel le métal du groupe VIII est le palladium.

4. Procédé selon l'une quelconque des revendications 1 à 3, dans lequel la teneur en métal du catalyseur est de 0,01 à 2,0% en poids.

5. Procédé selon l'une quelconque des revendications 1 à 4 dans lequel la zéolite est une zéolite d'aluminosilicate.

6. Procédé selon la revendication 5, dans lequel la zéolite d'aluminosilicate a un rapport molaire silice/alumine supérieur à 12 et un indice de contrainte de 1 à 12.

7. Procédé selon la revendication 6, dans lequel la zéolite est la ZSM—5.

8. Procédé selon la revendication 7, dans lequel la catalyseur est un mélange physique de ZSM—5 et de palladium sur support.